# EUROPEAN PATENT APPLICATION

(11) **EP 1 290 975 A1**
(43) Date of publication of application: **12.03.2003**
(21) Application number: 02256112.0
(22) Date of filing: 03.09.2002
(51) Int. Cl.: A61B 5/024, G04G 1/00

(54) **Pulsimeter**

(30) Priority: 06.09.2001 JP 2001270280
(71) Applicant: Seiko Instruments Inc., Chiba-shi, Chiba (JP)
(72) Inventor: Kato, Kazuo, c/o Seiko Instruments Inc., Chiba-shi, Chiba (JP)
(74) Representative: Sturt, Clifford Mark

(57) **Abstract**

There is provided a pulsimeter capable of stopping pulse rate measurement processing of the pulsimeter when the pulse sensor is not in contact with the skin or the pulse sensor is poorly attached. The pulsimeter of the present invention is characterized by including drive means for driving and stopping the pulse sensor, contact detection means for detecting whether or not the pulse sensor is in contact with a skin, and control means for stopping the drive of the pulse sensor by the drive means when it is detected by the contact detection means that the pulse sensor is not in contact with the skin.

## Description

Field of the Invention: The present invention relates to a portable pulsimeter to be equipped in, for example, a wristwatch or the like, and more specifically to a pulsimeter which does not measure a pulse rate when a pulse sensor is not brought into contact with a skin.

With growing interest in health care, a portable pulsimeter capable of measuring a pulse rate while attached to an arm or the like, for example, a wristwatch equipped with a pulsimeter (for example, see Japanese Patent Application Laid-open No. Hei 10-258040) is devised. According to such a portable pulsimeter, light or ultrasound is irradiated to a blood vessel, a reflected signal from the blood vessel is received using a pulse sensor including an optical sensor, and a pulse signal corresponding to a pulse is extracted from the reflected signal to obtain a pulse rate.

According to a conventional wristwatch equipped with a pulsimeter, when it goes into a pulse rate measurement mode by an input instruction from a user, the pulse sensor is driven by a driver circuit to perform a pulse detection operation regardless of whether or not the pulse sensor is attached to the arm or the like of the user.

Here, when the pulse sensor is not attached to the arm or the like of the user, since the reflected signal from the blood vessel becomes small, an internal microcomputer (for example, a CPU or the like) judges insufficient sensitivity of a receiving signal and increases an amplification factor of an amplifying circuit for amplifying an output signal from the pulse sensor in order to improve the sensitivity. Thus, the microcomputer increases stepwise the amplification factor of an amplifying circuit while the insufficient sensitivity is judged and the amplification factor finally reaches the maximum. After that, the pulse sensor continues the pulse detection operation at the maximum amplification factor.

However, according to such a conventional wristwatch equipped with a pulsimeter, when the pulse sensor is detached while the pulse rate measurement mode is set by a user, or when the pulse rate measurement mode is set by a user while the pulse sensor is detached, there is a problem in that an unnecessary pulse detection operation is performed and the utilization efficiency of a battery is reduced.

When the pulse sensor is a type for irradiating light or ultrasound to a skin and detecting a pulse signal, since the power consumption for irradiating light or ultrasound is relatively large, this problem becomes particularly remarkable.

Thus, this becomes a problem even in the wristwatch equipped with the pulsimeter. In addition, when it is a specific device for measuring a pulse rate and the detection operation is performed with a state in which the pulse sensor is not attached or is poorly attached, a reduction in utilization efficiency of a battery further becomes a problem.

The present invention has been made in view of the above problems, and an object of the present invention is therefore to provide a pulsimeter capable of stopping pulse rate measurement processing of the pulsimeter when the pulse sensor is not in contact with a skin.

In order to solve the above problems, a pulsimeter of the present invention is characterized by including drive means for driving a pulse sensor, contact detection means for detecting whether or not the pulse sensor is in contact with a skin, and control means for stopping drive of the pulse sensor by the drive means when the contact detection means detects that the pulse sensor is not in contact with the skin.

According to the present invention, the contact detection means detects whether or not the pulse sensor is in contact with the skin. When it detects that the pulse sensor is not in contact with the skin, the control means stops the drive of the pulse sensor by the drive means. Here, the contact with the skin includes not only the case of a direct contact with the skin but also a state in which there is a somewhat distance from the skin but it is considered that the sensor is substantially attached thereto.

Therefore, according to the present invention, when the pulse sensor is not in contact with the skin, since the drive of the sensor is stopped, the utilization efficiency of the battery of the pulsimeter can be improved.

Also, according to the present invention, the above structure may further include display means for displaying pulse data measured by the pulse sensor. When the contact detection means detects that the pulse sensor is not in contact with the skin, the display means may display a message that the pulse sensor is not in contact with the skin.

Further, according to the present invention, signal detection judging means for judging whether or not a pulse signal from the pulse sensor is detected may be further included. Even in the case where the contact detection means detects that the pulse sensor is in contact with the skin, when it is judged by the signal detection judging means that the pulse signal from the pulse sensor is not detected, the display means may be constructed so as to display a message that an attachment position of the pulse sensor is unsuitable. According to the present invention, since it is constructed so as to further include such display means, the convenience of a user can be improved.

Also, according to the present invention, the contact detection means may be constructed so as to include two electrodes protruding from the rear side of a cabinet with the pulse sensor and to detect whether or not the pulse sensor is in contact with the skin by a potential difference between the electrodes. The two electrodes of the contact detection means each protrude in a surrounding shape of the pulse sensor and it may be constructed such that the two electrodes are provided to be opposite to each other at a skin contact side of the cabinet. The surrounding shape includes a substantially semicircle or a substantially semisquare.

Further, according to the present invention, the contact detection means may be constructed so as to judge whether or not the pulse sensor is in contact with the skin based on a predetermined time. Thus, when it is judged based on the predetermined time, since there is no case where the drive of the pulse sensor is stopped by instant poor contact or the like, the pulse rate can be efficiently measured.

Also, according to the present invention, in any one of the above structures, it may be constructed as a pulsimeter in which the pulse sensor transmits and receives ultrasound.

A pulsimeter of the present invention is characterized by including a driver to drive a pulse sensor, a contact detector to detect whether or not the pulse sensor is in contact with a skin, and a controller to stop drive of the pulse sensor by the driver when the contact detector detects that the pulse sensor is not in contact with the skin.

Also, according to the present invention, further comprising a display to display pulse data measured by the pulse sensor.

Also, according to the present invention, wherein when the contact detector detects that the pulse sensor is not in contact with the skin, the display may display a message that the pulse sensor is not in contact with the skin.

Also, according to the present invention, further comprising a signal detection judging circuit to judge whether or not a pulse signal from the pulse sensor is detected, wherein even in the case where the contact detector detects that the pulse sensor is in contact with the skin, when it is judged by the signal detection judging circuit that the pulse signal from the pulse sensor is not detected, the display displays a message that an attachment position of the pulse sensor is unsuitable.

Embodiments of the present invention will now be described by way of further example only and with reference to the accompanying drawings, in which:-
Fig. 1 is a schematic block diagram showing a circuit structure of a pulsimeter according to one embodiment mode to which the present invention is applied;
Figs. 2 are concept views of pulse sensor module structures of the pulsimeter according to one embodiment mode to which the present invention is applied, in which 2A is a side view, 2B is a rear view, and 2C shows a modified example of an arrangement of respective elements in a rear surface;
Fig. 3 is a schematic diagram showing a circuit structure of a contact detection circuit according to one embodiment mode to which the present invention is applied;
Fig. 4 is a flowchart of pulse signal detection processing executed by a CPU according to one embodiment mode to which the present invention is applied; and
Fig. 5 is a flowchart of pulse rate measurement processing executed by a CPU according to one embodiment mode to which the present invention is applied.

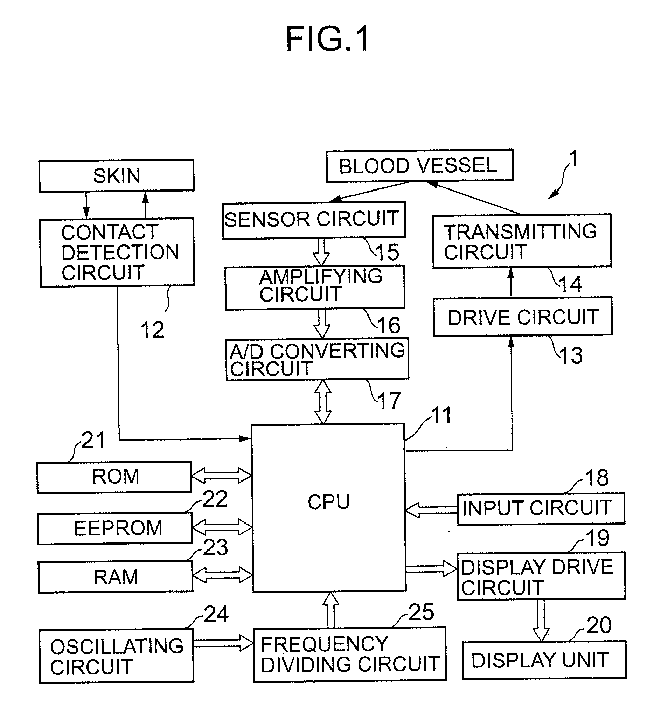

Hereinafter, an embodiment mode of a pulsimeter according to the present invention will be described in detail with reference to Figs. 1 to 5. Note that the embodiment mode is indicated as an example and the present invention should not be restrictively interpreted by this.

Fig. 1 is a schematic block diagram indicating a circuit structure of a pulsimeter 1 according to an embodiment mode to which the present invention is applied. The pulsimeter 1 includes a CPU (central processing unit) 11, a contact detection circuit 12, a drive circuit 13, a transmitting circuit 14, a sensor circuit 15, an amplifying circuit 16, an A/D converting circuit 17, an input circuit 18, a display drive circuit 19, a display unit 20, a ROM (read only memory) 21, an EEPROM (electrically erasable programmable read only memory) 22, a RAM (random access memory) 23, an oscillating circuit 24, and a frequency dividing circuit 25. The respective elements except for the transmitting circuit 14, the sensor circuit 15, the amplifying circuit 16, and the display unit 20 are connected with the CPU 11 through buses.

The CPU 11 executes pulse signal detection processing and pulse rate measurement processing, which are described later, and these processing programs are stored in the ROM 21. The input circuit 18 includes an input button which can be pushed down by a user. The RAM 23 expands the corresponding processing program stored in the ROM 21 at the execution of the above processing and temporally stores outputs based on the processing (for example, measurement results of the pulse rate and data indicating no contact).

Also, the CPU 11 causes the display unit 20 to display the outputs by the processing, which is temporally stored in the RAM 23, through the display drive circuit 19. Note that, when the pulsimeter 1 is a wristwatch equipped with a pulsimeter, the display unit 20 may be a liquid crystal display unit capable of performing digital display, which is located in a dial of the wristwatch.

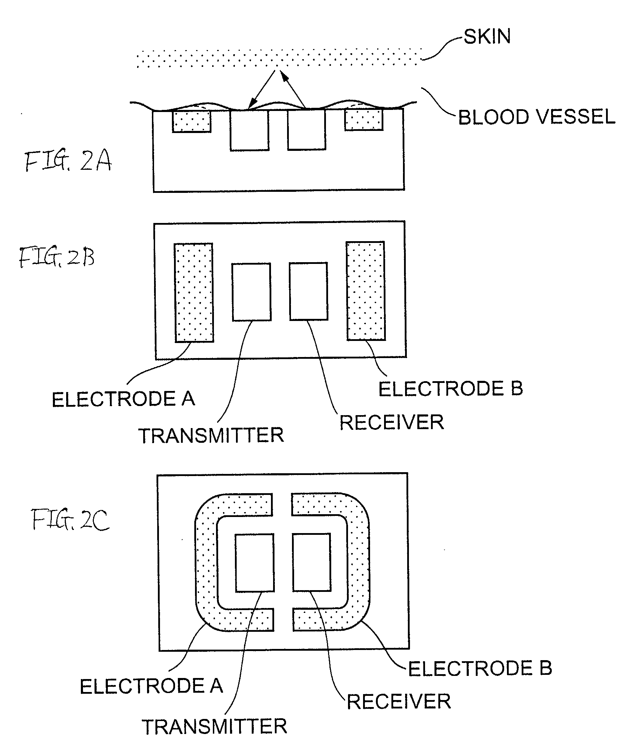

The contact detection circuit 12 is a circuit for detecting whether or not the pulsimeter 1 is in contact with the skin of the user in the pulse signal detection processing and includes two electrodes A and B as shown in Fig. 2. The drive circuit 13 drives or stops the transmitting circuit 14 by an instruction from the CPU 11. The transmitting circuit 14 includes a transmitter such as a light emitting diode for irradiating light to a blood vessel under the skin of the user. The transmitter may be a one for outputting ultrasound.

The sensor circuit 15 includes a receiver such as a photodiode for detecting light or ultrasound which is irradiated from the transmitting circuit 14 and reflected from the blood vessel as a reflected signal and outputs the reflected signal to the amplifying circuit 16. The amplifying circuit 16 amplifies an input signal inputted from the sensor circuit 15 at a predetermined amplification instructed by the CPU 11 and outputs it to the A/D converting circuit 17. In addition, the A/D converting circuit 17 A/D-converts the input signal and outputs digital data to the RAM 23 through the CPU 11.

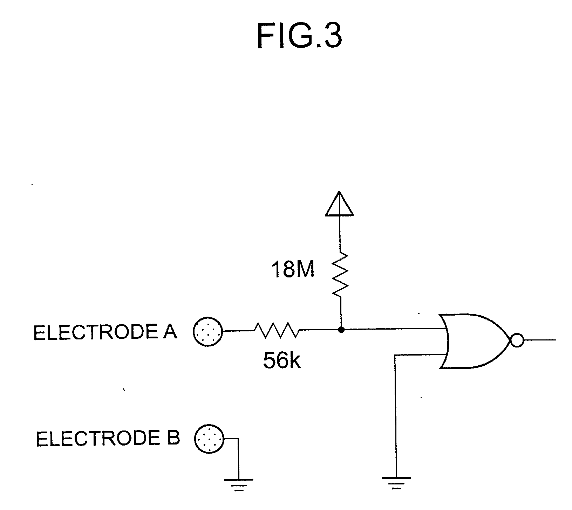

Figs. 2 schematically show pulse sensor module structures of the pulsimeter 1 of the present invention, 2A is a side view, 2B is a rear view, and 2C shows a modified example of a rear surface. In these drawings, with respect to the electrodes A and B included in the contact detection circuit 12, one becomes a ground (earth) of this circuit and the other is connected with the input circuit pulled up by a high resistor. Fig. 3 shows one example of electrode connection.

Fig. 3 shows a circuit structure of the two electrodes of the contact detection circuit 12 of the present invention. A resistor having a high resistance of 18 MΩ is used here. When both the two electrodes are in contact with the skin, a voltage between both the electrodes is decreased by discharge through the skin. When it is detected through a gate that the voltage is low (Low), the CPU 11 judges that the pulse sensor is in contact with the skin.

Also, the transmitter and the receiver of the pulse sensor are shown in Figs. 2. When light is irradiated to the skin, for example, a light emitting diode can be used as the transmitter and a photodiode can be used as the receiver. A pulse signal corresponding to a pulse is detected from the received light to measure a pulse rate. In contrast to this, it is utilized in the case of an ultrasound irradiation type that a pulse is a flowing blood flux. That is, the Doppler effect of the reflected signal produced when a blood vessel expands and contracts by a flowing blood is utilized and a change in a frequency of ultrasound is detected to measure a pulse rate.

Another embodiment mode of the present invention is indicated in Fig. 2C as a modified example of the arrangement of the respective elements shown in Fig. 2B. In this drawing, the pulsimeter 1 is constructed such that two electrodes protruding in a circumference shape of a semicircle are located to be opposite to each other and the transmitter and the receiver are provided inside the circumference. Thus, when the transmitter and the receiver are surrounded by the two electrodes, it can be prevented that the receiver is influenced by the disturbance of an external light at a pulse rate measurement, that is, light shielding can be conducted.

Note that the shape of the two electrodes is not limited to the above example. The two electrodes are preferably ones which are capable of preventing the influence of the disturbance of an external light on the receiver and have a shape and a size such that they are fit on the rear surface of the cabinet. However, in order to judge whether or not the pulse sensor is suitably in contact with the skin, these electrodes are preferably located at the outer side than at least the transmitter and the receiver.

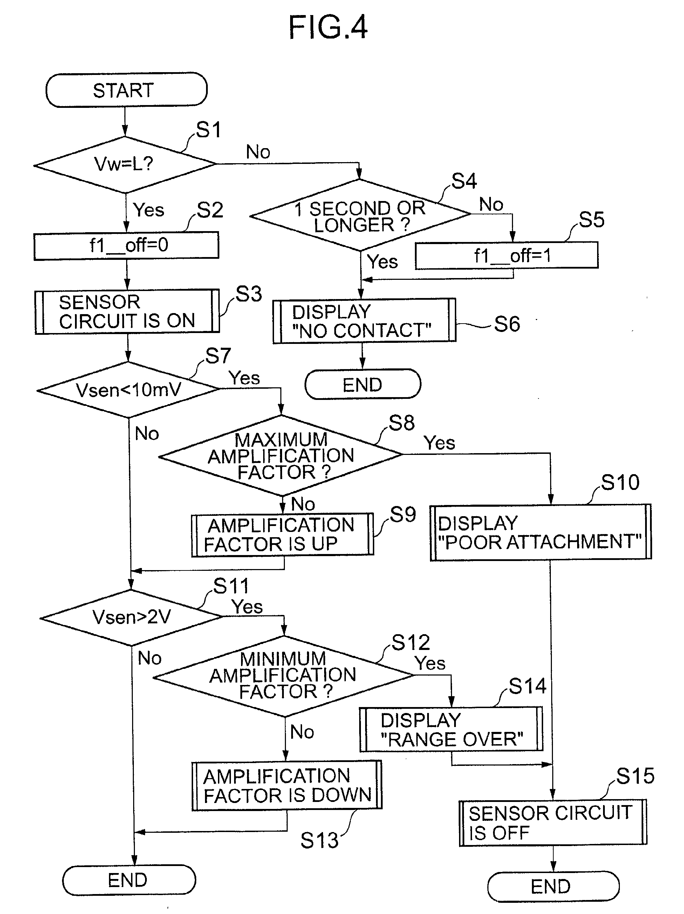

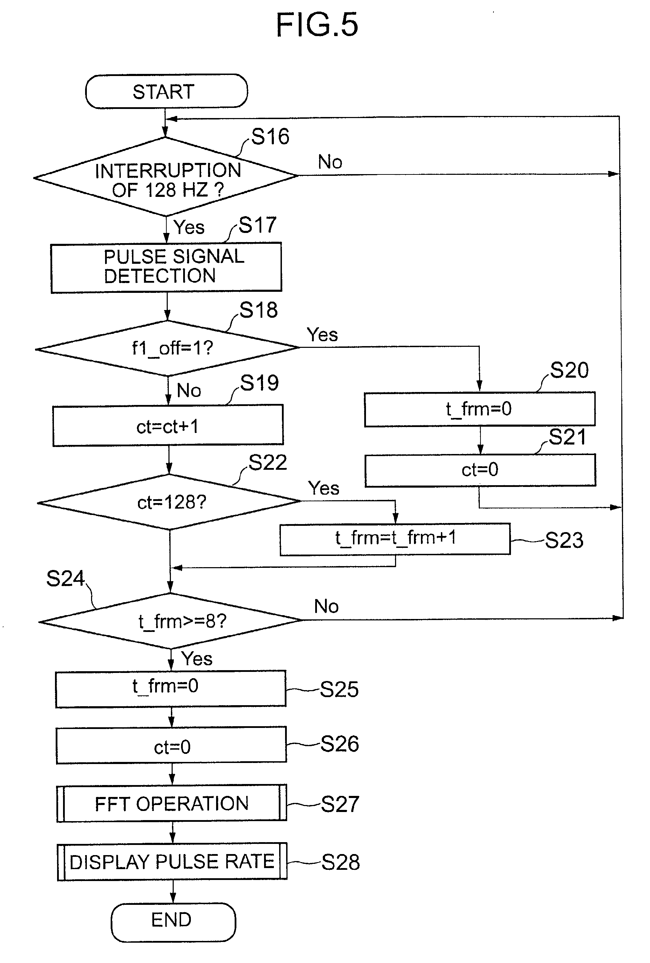

Next, the operation of the pulsimeter 1 of the embodiment mode will be described using flowcharts shown in Figs. 4 and 5. First, pulse signal detection processing of the pulsimeter 1 will be described. Fig. 4 is a flowchart of the pulse signal detection processing according to one embodiment mode to which the present invention is applied. The pulse signal detection processing is started in accordance with an input instruction (corresponding to interruption of 128 Hz in the pulse rate measurement processing) from the input circuit 18 by a user.

In step S1, the CPU 11 judges whether or not a potential difference Vw between the electrodes, which is detected by the contact detection circuit 12 is Low. When it is Low, the CPU 11 sets f1_off = 0, causes the RAM 23 to store this data (step S2), and causes the transmitting circuit 14 and the sensor circuit 15 to be in an ON state through the drive circuit 13. Then, the processing is transferred to step S7.

On the other hand, when Vw is not Low, the processing is transferred to step S4 and the CPU 11 judges whether or not this state is continued for 1 second or longer. Here, when the state where Vw is not Low is continued for 1 second or longer, the CPU 11 judges that the pulse sensor is not in contact with the skin. When the state is continued for 1 second or shorter, the state where the sensor is instantaneously floated is judged. However, a duration for contact detection is preferably set to be in a range in which there is not a problem such that a user is kept waiting long enough to feel discomfort at usage, and so forth. It may be set not only 1 second but also 2 to 3 seconds.

In step S4, when it is judged that the state is not continued for 1 second or longer, the CPU 11 sets f1_off = 1 and causes the RAM 23 to store this data (step S5). When it is judged that the state is continued for 1 second or longer, processing is not performed. Then, the CPU drives the display drive circuit 19 to display a message "no contact" on the display unit 20 (step S6) and the pulse signal detection processing is completed.

In step S7, CPU 11 judges whether or not an output voltage Vsen of the sensor circuit 15 is smaller than 10 mV. When it is judged that the voltage is smaller than 10 mV, the CPU 11 judges whether or not an amplification factor of the amplifying circuit 16 is the maximum in step S8. When the amplification factor is not the maximum, it is upped (step S9) and the processing is transferred to step S11. On the other hand, when the amplification factor is the maximum, the CPU 11 drives the display drive circuit 19 to display a message "poor attachment" on the display unit 20 (step S10) and causes the pulse sensor to be in an OFF state by the drive circuit 13 (step S15) and the pulse signal detection processing is completed.

On the other hand, when it is judged in step S7 that Vsen is not smaller than 10 mV, the CPU 11 judges whether or not Vsen is larger than 2 V in step S11. When it is judged that Vsen is larger than 2 V, the CPU 11 judges whether or not the amplification factor of the amplifying circuit 16 is the minimum in step S12. When the amplification factor is not the minimum, it is downed (step S13) and the pulse signal detection processing is completed. On the other hand, when the amplification factor is the minimum, the CPU 11 drives the display drive circuit 19 to display a message "range over" on the display unit 20 (step S14) and causes the pulse sensor to be in an OFF state by the drive circuit 13 (step S15) and the pulse signal detection processing is completed. On the other hand, when it is judged that Vsen is not larger than 2 V, the pulse signal detection processing is completed without any processing.

Note that, in order to avoid a repetition of descriptions, one stage loop is formed with respect to the up and down of the amplification factor of the amplifying circuit 16. However, in order to conduct further accurate judgement by the CPU 11, multistage control is required.

Next, the pulse rate measurement processing of the pulsimeter 1 will be described. Fig. 5 is a flowchart of pulse rate measurement processing according to one embodiment mode to which the present invention is applied. In step the CPU judges whether or not an interruption of 128 Hz is produced and waits in step S16 until the interruption is produced. When the interruption is produced, the CPU 11 causes the RAM 23 to expand the corresponding program stored in the ROM 21 and executes the above pulse signal detection processing (step S17). Then, the processing is transferred to step S18.

In step the CPU 11 judges whether or not f1_off stored in the RAM 23 is 1. When f1_off is 1, the CPU 11 sets t_frm = 0 and ct = 0 (steps S20 and S21) and the processing is returned to step S16. On the other hand, when f1_off is not 1, the CPU 11 sets ct = Ct + 1 (step S19) and the processing is transferred to step S22.

In step S22, the CPU 11 judges whether or not ct is 128. When ct is 128, the CPU 11 sets t_frm = t_frm + 1 (step S 23). When ct is not 128, the processing is transferred to step S24 without any processing.

In step S24, the CPU 11 judges whether or not t_frm is 8 or larger. When t_frm is smaller than 8, the processing is returned to step 16 and the above processing is repeated by the CPU 11. When t_frm is 8 or larger, the CPU 11 sets t_frm = 0 and ct = 0 (steps S25 and S26) and executes first Fourier transform (FFT) processing based on those set values. Then, the CPU 11 drives the display drive circuit 19 to display a measured pulse rate on the display unit 20 (step S28) and the pulse rate measurement processing is completed.

As described above, according to the embodiment mode, the pulsimeter 1 is constructed by including the drive circuit 13 for driving the transmitting circuit 14, the contact detection circuit 12 for detecting whether or not the pulse sensor is in contact with the skin, the CPU 11 for controlling these circuits, and the ROM 21 for storing the respective processing programs. In such a structure, when it is detected that the pulse sensor is not in contact with the skin, the CPU 11 stops the drive of the pulse sensor by the drive circuit 13. Even when it is detected that the pulse sensor is in contact with the skin, if the output of the sensor circuit 15 has an insufficient sensitivity, the CPU judges whether or not the pulse sensor is poorly attached in accordance with the amplification factor of the amplifying circuit 16 and stops the drive of the pulse sensor by the driver circuit 13 based on the judgement. As a result, the utilization efficiency of the battery of a pulsimeter can be improved by using the plusimeter 1.

According to the pulsimeter of the present invention, when the pulse sensor is not in contact with the skin or when it is poorly attached, since the drive of the sensor can be stopped, the utilization efficiency of the battery of the pulsimeter can be improved.

## Claims

1. A pulsimeter comprising:
drive means for driving a pulse sensor,
contact detection means for detecting whether or not the pulse sensor is in contact with a skin, and
control means for stopping drive of the pulse sensor by the drive means when the contact detection means detects that the pulse sensor is not in contact with the skin.

2. A pulsimeter according to claim 1, further comprising display means for displaying pulse data measured by the pulse sensor.

3. A pulsimeter according to claim 2, wherein when the contact detection means detects that the pulse sensor is not in contact with the skin, the display means may display a message that the pulse sensor is not in contact with the skin.

4. A pulsimeter according to claim 2, further comprising:
signal detection judging means for judging whether or not a pulse signal from the pulse sensor is detected,
wherein even in the case where the contact detection means detects that the pulse sensor is in contact with the skin, when it is judged by the signal detection judging means that the pulse signal from the pulse sensor is not detected, the display means displays a message that an attachment position of the pulse sensor is unsuitable.

5. A pulsimeter according to claim 1, wherein the contact detection means includes two electrodes provided at a side where a cabinet having the pulse sensor is in contact with the skin and detects whether or not the pulse sensor is in contact with the skin by a potential difference between the electrodes.

6. A pulsimeter according to claim 5, wherein the two electrodes of the contact detection means each protrude in a shape of surrounding the pulse sensor, and
wherein the two electrodes are provided to be opposite to each other at a skin contact side of the cabinet.

7. A pulsimeter according to claim 1, wherein the contact detection means judges whether or not the pulse sensor is in contact with the skin based on a predetermined time.

8. A pulsimeter according to claim 1, wherein the pulse sensor measures pulse rate by transmitting and receiving ultrasound.

9. A pulsimeter comprising:
a driver to drive a pulse sensor,
a contact detector to detect whether or not the pulse sensor is in contact with a skin, and
a controller to stop drive of the pulse sensor by the driver when the contact detector detects that the pulse sensor is not in contact with the skin.

10. A pulsimeter according to claim 9, further comprising a display to display pulse data measured by the pulse sensor.

11. A pulsimeter according to claim 10, wherein when the contact detector detects that the pulse sensor is not in contact with the skin, the display may display a message that the pulse sensor is not in contact with the skin.

12. A pulsimeter according to claim 10, further comprising:
a signal detection judging circuit to judge whether or not a pulse signal from the pulse sensor is detected,
wherein even in the case where the contact detector detects that the pulse sensor is in contact with the skin, when it is judged by the signal detection judging circuit that the pulse signal from the pulse signal is not detected, the display displays a message that an attachment position of the pulse sensor is unsuitable.
